# EUROPEAN PATENT SPECIFICATION

(11) **EP 1 506 027 B1**
(45) Date of publication and mention of the grant of the patent: **13.09.2006**
(21) Application number: 03741809.2
(22) Date of filing: 22.05.2003
(51) Int. Cl.: A61L 27/38, A61L 27/50, A61K 49/04

(54) **CELL DELIVERY FLUID FOR PREVENTION OF CELL SETTLING IN DELIVERY SYSTEM**
ZELLABGABEFLÜSSIGKEIT ZUR VERHINDERUNG VON ZELLABLAGERUNGEN IN ABGABESYSTEMEN
FLUIDE DE DISTRIBUTION CELLULAIRE PERMETTANT DE PREVENIR LA SEDIMENTATION DANS UN SYSTEME DE DISTRIBUTION

(30) Priority: 22.05.2002 US 382764 P; 21.05.2003 US 442525
(43) Date of publication of application: 16.02.2005
(73) Proprietor: MEDTRONIC, INC., Minneapolis, MN 55432 (US)
(72) Inventor: MORRIS, Mary, M., Mounds View, MN 55112 (US); FERNANDES, Brian, C., A., Roseville, MN 55113 (US); SPEAR, Stanton, C., Arden Hills, MN 55112 (US); BERGAN, Matthew, A., Brooklyn Park, MN 55445 (US); GARDESKI, Kenneth, C., Plymouth, MN 55442 (US)
(74) Representative: Hughes, Andrea Michelle
(86) International application number: PCT/US2003/016156
(87) International publication number: WO 2003/099347

(56) References cited:
- WO-A-00/21577
- WO-A-02/085325
- US-A- 5 543 316
- US-A- 5 667 778
- US-A- 6 129 761
- US-B1- 6 171 610

## Description

### FIELD OF THE INVENTION

The invention relates to a medical device-related cell delivery carrier medium, and methods of manufacturing pharmaceutical compositions for delivering cells for generating tissue growth.

### BACKGROUND OF THE INVENTION

Previously, there has been interest in the delivery of cells to locations within mammalian bodies to effect new growth of tissue. This technology is designed to promote growth of new tissue from implanted cells, often originating from the same mammal receiving the cells, and designed to generate new tissue in the region of implantation. Various types of tissue may be implanted, including for example, bone, cartilage, muscle and other types.

Cardiac tissue has also been the subject of cell delivery efforts in order to repair cardiac walls and other regions severely damaged by myocardial infarctions or congestive heart failure. One example of such use includes skeletal muscle-derived myoblasts or stem cells delivered surgically into the myocardia of the patient to regenerate damaged tissue, promote revascularization and angiogenesis. Desired volume concentrations of cells per delivery vary according to indications, but it is not uncommon to have tens to hundreds of millions of cells intended to be delivered to one or more sites.

### BRIEF SUMMARY OF THE INVENTION

The invention involves the recognition of the problem of cells settling during the attempted delivery of cells to designated sites thus preventing the intended concentrations of cells from being delivered. A method of overcoming this problem, and subsequent delivery inaccuracies, is to provide a method of preparing a cell-carrier liquid suspension for implantation into a mammal which prevents cell settling. The method involves selecting a type of cell for implantation into a mammal and identifying the specific gravity of the cell type. Then a carrier liquid is selected having a specific gravity within a range of specific gravities. This enables an approximate match of the specific gravity with the selected cell type such that the cells do not tend to float or settle, but stay in suspension to allow delivery of an acceptable ratio of cells at one or more delivery destinations in the mammal. It is further desired to ensure that the identified liquid has an appropriate osmolality and pH to ensure an acceptable viability ratio of the cells at the delivery destination. By mixing the carrier liquid and the cells into a proper liquid suspension, it is possible to achieve a more accurate and consistent delivery of the intended amount of specific cells at a specific site- either percutaneously or surgically.

A cell carrier liquid suspension for delivery into tissue of a mammal is also provided in which the density of a carrier fluid component is matched to the density of the cells of interest. Accordingly, the cell carrier liquid suspension provides a tissue forming liquid suspension with greater efficacy in treating defective tissue.

### BRIEF DESCRIPTION OF THE DRAWINGS

Figure 1 is a graph of top and bottom cell count ratios.
Figure 2 is a graph of a ratio of top and bottom total cell counts.
Figure 3 is a graph of myoblast cell settling.
Figure 4 is a table of catheter values.
Figure 5 consists of images taken during cell settling.
Figure 6 is a table of delivery dynamics for cell delivery through catheters.
Figures 7A-H are SEM photographs of the inner lumens of catheters.

### DETAILED DESCRIPTION OF THE INVENTION

The invention provides new methods of manufacturing compositions for accurate, predictable delivery of cells in suspension to a targeted delivery site within a mammal. The methods and compositions contemplate delivery of a more accurate and precise amount of cells and suspension liquid in order to grow new tissue at the target locations. Such cells may include mature myogenic cells (e.g., skeletal myocytes, cardiomyocytes, purkinje cells, fibroblasts), progenitor myogenic cells (such as myoblasts), mature non-myogenic cells (such as endothelial and epithelial cells), hematopoietic cells (monocytes, macrophages, fibroblasts, alpha islet cells, beta islet cells, cord blood cells, erythrocytes, platelets, etc.) or stem cells (pluripotent stem cells, mesenchymal stem cells, endodermal stem cells, ectodermal stem cells, whether adult or embryonic, or whether autologous, allogenic, or xenogenic). More particularly, cells which may be delivered according to this invention further include islet cells, hepatocytes, chondrocytes, osteoblasts, neuronal cells, glial cells, smooth muscle cells, endothelial cells, skeletal myoblasts, nucleus pulposus cells, and epithelial cells. Any of the mentioned cell types can be genetically engineered to contain DNA or RNA introduced into the cell by recombinant techniques. The DNA or RNA introduced into the cell make include, but are not limited to, new genes, promoters, interfering RNAs, and the like. Accordingly, tissue may be formed resulting in new growth of cartilage, bone, skin, epithelial layers, new organs, central nervous system tissue, and muscle- including that tissue appropriate for re-generation of certain cardiac function. It is recognized that the methods and compositions, along with the essential aspects of the invention, may allow delivery of numerous types of cells whether listed above, by example, or not, in a much improved manner and with increased efficacy. It is recognized that any of the above mentioned cells used in conjunction with the cell density matched carrier fluids may be labeled or tagged with radio-isotope labels, fluorescently tagged, enzymatically tagged, or further used in combination with specific cell protein antibody markers.

The challenge of accurate cell delivery (and accompanying nutrients) has been addressed by use of a gel matrix (or similar structures) as a carrier medium, such as shown in U.S. Patents 6,171,610 B1; 6,129,761; 5,667,778. Typically, the cells are placed in suspension in hydrogels in order to stabilize the cells at the delivery site. However, problems arise from use of the gels due to the relatively high pressure required to inject the high viscosity gels via a catheter, which is generally considered a less invasive approach than a direct surgical path. Moreover, gels utilize material having relatively longer molecules and higher viscosities that causes increased shear stress during delivery resulting in more cell damage. Alternatively, gels delivered surgically or endoscopically must also share the risk factors and inefficiencies attendant to those procedures.

Another type of cell delivery medium is that shown in U.S. Patent 5,543,316, which describes an injectable composition comprising myoblasts and an injectable grade medium having certain components designed for maintaining viability of the myoblasts for extended periods of time. The osmolality of the medium is preferably from about 320 mOsm/kg to about 550 mOsm/kg (e.g, more preferably selected from the osmolality of about 250 mOsm/kg, about 300 mOsm/kg, about 350 mOsm/kg, 400 mOsm/kg, about 450 mOsm/kg, about 500 mOsm/kg, about 550 mOsm/kg, about 600 mOsm/kg, and the like). This technique, combined with attempted delivery of very high concentrations of cells, represents another method of overcoming the challenges of effective cell delivery therapy.

The above reference is one example of the past misunderstanding regarding the cause of cell delivery inaccuracies. In the past it has been assumed that cell death was the cause of cell delivery problems, such as that caused by shear stress induced by a combination of time, pressure, diameter of delivery vehicle lumen, and the size of the cells being delivered. What was not realized was that cell settling in liquid solutions was an important cause of delivery inaccuracies.

What has not been realized or identified is a simple solution to the problem of inconsistent concentrations of cells being delivered in a liquid medium. Applicants have recognized this problem and identified a cell delivery medium having characteristics designed to overcome this obstacle to effective therapy. The cell delivery medium is density matched with the cells it is delivering. For example, in a transvenous catheter delivery, it is preferred that the cell delivery medium has a low enough viscosity to be deliverable at pressures of less than about 689 kPA (100 psi) (including but not limited to such pressures as about equal to or less than 552 kPa (80 psi), 483 kPa (70 psi), 414 kPa (60 psi), 276 kPa (40 psi), 241 kPa (35 psi), 138 kPa (20 psi) or 103 kPa (15 psi) and the like).

One skilled in the art recognizes that the internal diameter (I.D.) of the delivery tube affects the fluid dynamics of delivered solutions. For example, in a 0.305 mm (0.012 inch) inner diameter, 1.52 m (60 inch) length catheter it was possible to readily deliver a I centipoise fluid but not a 5 centipoise fluid at the pressures used. In a similar example, in a 0.432 mm (0.017 inch) inner diameter, 0.305 m (12 inch) length catheter it was possible to readily deliver fluids to up to and including 50 centipoise. These characteristics will optimize cell viability, ease of physician delivery, and patient comfort and recovery.

It is recognized then that various internal diameters of catheters can be used with selected cell density solutions (including, but not limited to 0.432 mm (0.017 in.), 0.406 mm (0.016 in.), 0.356 mm (0.014 in.), 0.343 mm (0.0135 in.), 0.305 mm (0.0012 in.), 0.229 mm (0.009 in.) and the like). Similarly, because of the high survivability rate demonstrated for cells in these solutions, much higher shear rates can be used than previously believed possible, including but not limited to rates equal to or greater than 1000 l/sec, 2000 l/sec, 3000 l/sec, 4000 l/sec, 5000 l/sec, 6000 l/sec, 7000 l/sec, 8000 l/sec. One feature of the described cell delivery fluids is that they permit cells to survive much higher shear stress in catheters (including but not limited to equal or greater than 1 N/m², 2 N/m², 3 N/m², 4 N/m², 5 N/m², 6 N/m² and the like). One skilled in the art would recognize that the survivability of cells is proportional to the shear stress in the catheter and the length of time it experiences the effective shear forces. It is recognized that the effective time that time a cell experiences an effective shear stress in the catheter may be as short as about 10 msec to upward of 5000 msec (including ranges of less then 4000 m sec, less then 3000 msec, less then 2000 msec, less then 1000 msec.) Therefore, ideal survival rates for cells may be optimized by effectively matching the delivery requirements, the shear stress, and the delivery time.

It is also recognized that higher viscosities may be possible with cell delivery devices via a more direct surgical approach in delivery devices of relatively shorter length and possibly of a larger lumen size, and still enjoy the benefits of this invention. However, a cell carrier liquid medium must be density (or specific gravity) matched with the cells it is transporting for optimal results. However, the present invention may use less than optimally matched cell density carriers where the use of these carriers with the delivered cells improves at least one measurable fluid dynamic in the catheter or at least one measure of effective delivery. Consistent with the foregoing matching of density carriers is that the cell density solution may be within about 10%, within about 5%, within about 2%, within about I %, within about 0.1%, or within about 0.01 % of any given cell density. Known examples of media which may be appropriate, with proper formulation, include Isovue brand image enhancing media (sold by Bracco Diagnostics), perfluorooctyl bromide (perflubron), known under the Oxygent brand name (sold by Alliance Pharmaceuticals), dextran solutions, such as Dextran 40 I.P, Microspan 40 in normal saline, and MICROSPAN40 in 5% dextrose (manufactured by Leuconostoc mesenteroides).

In another embodiment of the present invention the cell density matched solutions may also serve as image enhancing agents. Image enhancing agents can be effectively used for imaging the delivered fluid and for balancing cell density. Such reagents include iodine based solutions (such as iopamidol, sold as Isovue-300 by Bracco Diagnostics), gadodiamide (Ominiscan sold by Claris Life Sciences), and InFeD (iron dextran manufactured by Schein Pharmaceutical).

In yet another embodiment of the present invention the cell density matched solutions may also be labeled or tagged to aid in its detection. A number of the mentioned reagents are amenable to being synthesized with radioactive elements or radioactively tagged (e.g, radio C¹⁴ or H³ labeled glucose solutions, radioactive Isovue, radio active iodine (I¹²⁵) regents, and the like). Image enhancing agents can be effectively used for imaging the delivered fluid and for balancing cell density. Such reagents include iodine based solutions (such as iopamidol, sold as Isovue-300 by Bracco Diagnostics), gadodiamide (Ominiscan sold by Claris Life Sciences), and InFeD (iron dextran manufactured by Schein Pharmaceutical).

A number of references are available for determining cell density. Listed below are some published density values for the cell types given:

**Cell Specific Gravities**

| | |
|---|---|
| red blood cells: | 1.10 |
| stem cells (CD34 cells): | 1.065 |
| platelets: | 1.063 |
| monocytes: | 1.068 |
| lymphocytes: | 1.077 |
| hepatocytes | 1.07-1.10 |
| granulocytes | 1.08-1.09 |

The listed values are given to be exemplary and not limiting, and can be experimentally determined for the relevant cell population. For example, Percoll (a product of Pharmacia) is a well referenced medium for density gradient centrifugation of cells. Percoll will form self-generated gradients during centrifugation so that cells mixed with Percoll prior to centrifugation will band isopycnically as the gradient is formed in situ. Percoll can be used with density marker beads (Sigma product # DMB-10) which can be used to find cell densities.

The cells delivered suspended in the described carriers may vary widely in the actual effective cell concentration. The cell concentration may vary from about 1x10⁹ cells per milliliter to about 1x10⁶ cells per milliliter (ml) (including from about 1x10⁹ cells/ml, about 5x10⁸ cells/ml, about 1x10⁸ cells/ml, about 5x10⁷ cells/ml, about 1x10⁷ cells/ml, about 5x10⁶ cells/ml, about 1x10⁶ cells/ml, and the like depending on cell size). Choice of the delivered concentration of cells along with the number of cells is one criteria matched in selecting the appropriate delivery carrier for the delivered cells and medium to the target site.

One of several goals of the carrier medium of this invention is to mitigate or prevent undesired settling of the cells placed in the carrier medium. This is done in order to achieve a known, consistent (and preferably very high) cell delivery concentration ratio, i.e., delivered cells as compared with available cells intended to be delivered by the physician to a specific site should be close to the value of 1:1. It is a similar goal to ensure that an acceptable viability ratio (preferably also near 1:1) is achieved by which a high percentage of delivered cells are functional and replicate at well accepted levels. Providing methods and compositions which achieve this goal permits vast improvement over the known delivery capabilities of this type of treatment and improves the reliability of this form of medical treatment available to millions of people.

Applicants realized through investigation that cell loss, rather than cell death, was possibly the critical issue in catheter-based cell delivery. Following that realization, certain experiments validated cell loss as being caused by cell settling rather than adhesion to the delivery structure. Applicants also realized that through investigation the issue of cell settling is a critical component to any cell delivery method whether through use of pumps or catheters. Catheter applications would include use with cardiac delivery catheters; epicardial, endocardial, pericardial, intralumenal (e.g., intracoronoary, intravenous) and the like. Use of cell density balanced solutions also would be appropriate in other non-cardiac delivery methodologies, such as neurovascular, peripheral vascular, and the like, and more generally to syringe delivery of cells.

### Example I

Fibroblast cells were stored in 50 mL centrifuge tubes over a period of 100 minutes, both on ice and at room temperature (RT). Samples were removed by pipette from the top and bottom of both the ice and RT suspensions every 20 minutes. No mixing was done for the first 60 minutes. At the 80 minute time point, gentle mixing (hand swirling) was done immediately before sampling. At the 100 minute time, hard mixing (vigorous hand swirling) was done immediately before sampling.

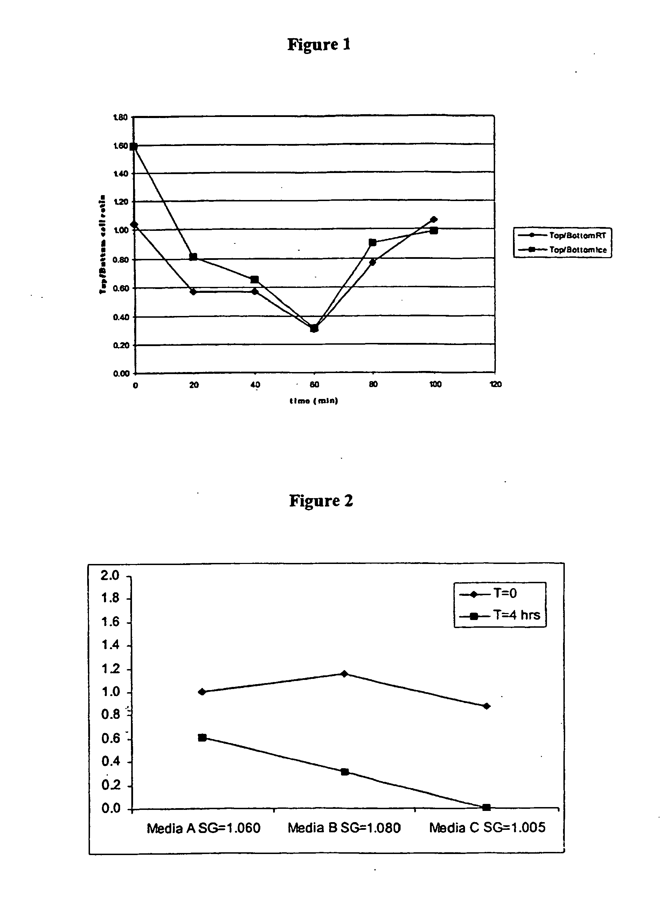

Figure 1 illustrates the top/bottom cell count ratios as the results of this experiment. The stratification of a static fibroblast suspension, whether kept at room temperature or on ice, is clearly demonstrated. By 60 minutes, the cell concentration taken from the top of the suspension was only 30% of that taken from the bottom. But after a gentle mix (the 80 minute time point), suspension equilibrium was clearly restored.

The results shown in Example I demonstrate that fibroblast suspensions do not maintain their initial concentration when allowed to sit over time. The results suggest that settling of the suspensions is occurring, but that even gentle mixing brings these suspensions back into equilibrium. This finding has potential impact on delivery device, delivery medium, and overall delivery system design, as it will be critical to assure that the appropriate concentration of therapeutic cells can be delivered through the catheter repeatedly and reliably. However, recognizing that rapid settling may occur, then constant agitation of a delivery vehicle and injectate medium may be necessary to prevent such phenomenon. But as a practical matter such agitation is not desirable by the physician. Consequently, Applicants identified a new solution to achieve a matched density of the carrier medium with the cells being delivered by that medium.

Prevention of human fibroblast settling was investigated using isotonic diluted solutions of Isovue brand image enhancing media to increase the specific gravity of the cell media above normal saline.

### Example II

A cell delivery medium was density matched with cells as a dilution of Isovue-300 image enhancing media (sold by Bracco Diagnostics) and human dermal fibroblasts. Isovue is a non-ionic image enhancing media with the active agent of iopamidol. The package insert for Isovue-300 lists the concentration as 300 mg/mL (61%), osmolality of 616 mOsm/kg water, viscosity at 20 C as 8.8 cP, and specific gravity of 1.339. Isovue-300 was then diluted 1:2 v/v (1 part Isovue to I part deionized water). The 1:2 diluted Isovue osmolality is about 300 mOsm/kg, and the calculated specific gravity is 1.170. The fibroblasts suspended in Hanks Balanced Salt Solution (HBSS) were then diluted with the diluted Isovue media to achieve a specific gravity of 1.060. Since the osmolality of both HBSS and diluted Isovue media is about 300 mOsm/kg, the dilution does not change the osmolality of the diluted cell suspensions.

The specific gravities of the solutions tested were 1.060 (Media A), 1.080 (Media B), and 1.005 (control- Media C). The cell concentrations on the top and bottom of the three solutions were counted before and after 4 hours of settling time. As shown in Figure 2, both the high density solutions significantly slowed the fibroblasts settling compared to the control. A comparison of top layers over time can also be made. After 4 hours, the control had zero cells in the top layer, and the diluted Isovue solutions had 105 & 57% of the initial cell count in the top layer. After 4 hours, the bottom layer for all the solutions contained more cells than counted initially.

Trypan cell counts (using Trypan Blue solution from Sigma Chemical) were performed at the two time points (0 & 4 hours). The number of stained (dead) cells were not significantly different for the diluted Isovue solutions compared to the saline control. Diluted Isovue media did not appear to significantly rupture cell membranes after 4 hours of contact. The cell proliferation assay indicated the fibroblasts proliferated after exposure to Isovue as well as with the saline control.

### Example III

This experiment was very similar to that performed in Example II, except that myoblasts were used rather than fibroblasts. Prevention of myoblast settling was investigated using isotonic diluted solutions of Isovue image enhancing media to increase the specific gravity of the cell media above normal saline. The specific gravities of the solutions tested were 1.060 (Media A), 1.080 (Media B), and 1.005 (control-Media C). The cell concentrations on the top and bottom of the three solutions were counted before and after 4 hours of settling time.

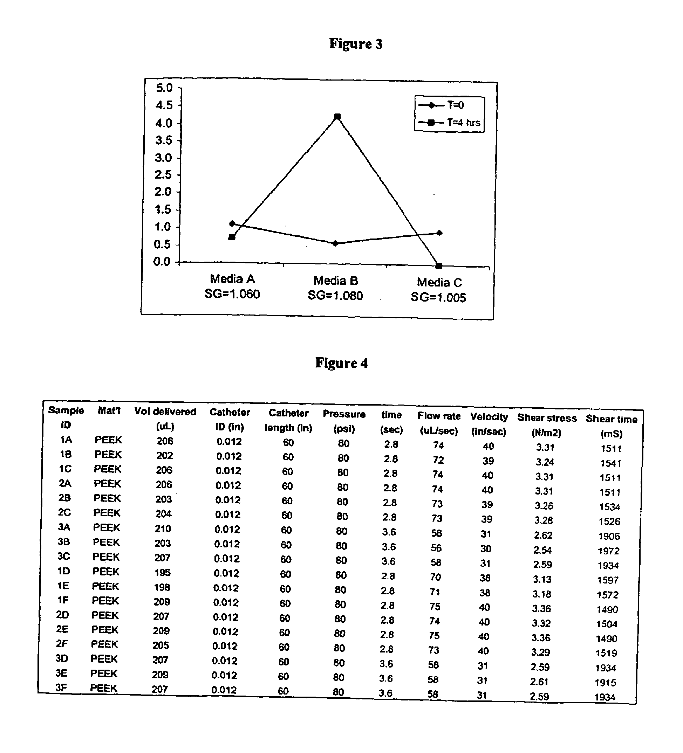

As shown in Figure 3, both the high-density solutions significantly slowed the myoblasts settling compared to the HBSS control. After 4 hours, the control had zero cells in the top layer, and the diluted Isovue solutions had 75 & 85% of the initial cell count in the top layer. After 4 hours, media A& B had strands of cells suspended with the control having some of the cells clumped on the bottom layer of the centrifuge tube. Strands of myoblasts after four hours were unexpected, as this was not observed with fibroblasts. The centrifuge tubes were gently mixed by hand swirling prior to the proliferation assay. These cell counts, after gentle mixing, indicate 24-40% of the cells were lost after four hours due to adherence to the vessel wall or each other (clumping). Cell settling was a more significant issue than adherence as the control had a 29-fold increase of cells on the bottom of the tube after four hours of settling. The density of the myoblasts is approximately 1.06 g/mL. The number of Trypan stained (dead) cells were very few and not significantly different for the three solutions. Diluted Isovue media does not appear to significantly rupture the myoblast cell membranes after 4 hours of contact. The cell proliferation assay indicates the myoblasts proliferated as well after exposure to Isovue as prior to exposure.

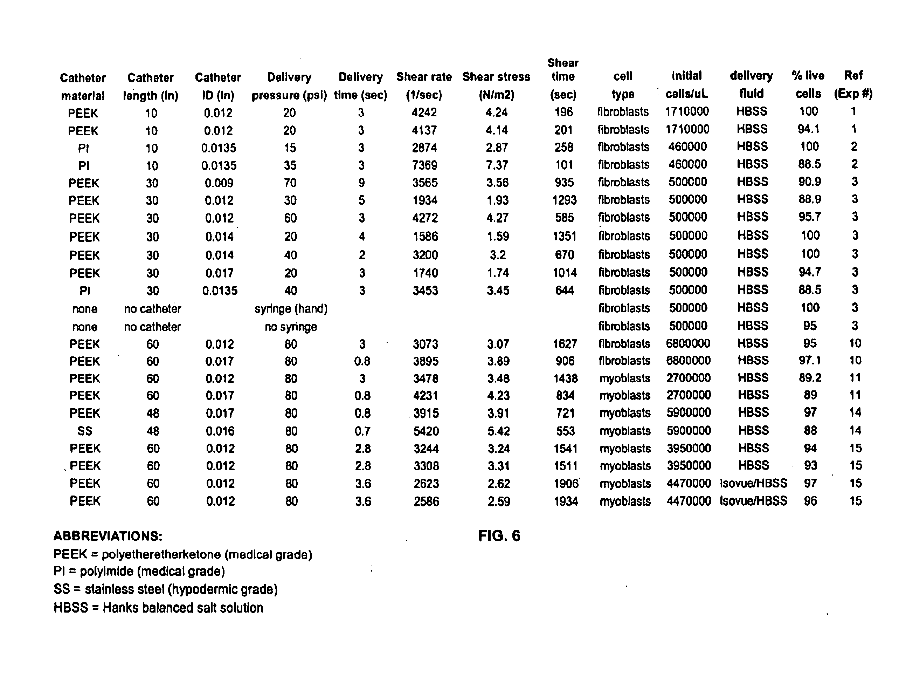

Applicants' previous experiments demonstrated that by matching the density and osmolality of a carrier fluid to that of the cells being delivered, then settling of cells can be minimized. Further experiments were done in view of the known problem of possible damage to cells due to adhesion with various materials. Although Applicants successfully performed
experiments which verified that viable, proliferative myoblasts can be delivered consistently through a wide range of catheter materials. Catheter materials may include various polymers, including but not limited to, poly etheretherketone (PEEK), polyimide (PI - medical grade), polyurethanes, polyamides, silicones, polyethylenes, polyurethane blends, polyether block amides (e.g., PEBAX), and the like, or including various metal materials, including but not limited to stainless steel (SS), titanium alloys, nickel titanium alloys (e.g. Nitinol), chromium alloys (MP35N, Elgiloy, Phynox, etc.), cobalt alloys, and the like. More preferably the catheter materials are chosen from the group of poly etheretherketone (PEEK), polyimide (PI), and stainless steel (SS). One feature studied when cells were delivered through catheters (after proper mixing in the cell density solution) was the behavior of myoblasts and fibroblasts in the various vessels used to hold, transport, and inject cells over the expected implant period. Accordingly, further experiments included combinations of density matched and non-density matched delivery media in catheters of lengths, lumen sizes, and materials representative of those suitable for transvenous cell delivery. As shown in Figures 4 and 6, and used in the investigations of Examples IV and VI below, some of the media experience relatively high shear time, which was previously believed to be a key indicator of adhesion and cell delivery inaccuracies.

Example IV evaluates the two technologies of cell delivery and prevention of cell settling performed simultaneously by delivering myoblasts through catheters using cell settling prevention media. The investigation focuses on the variation of parameters and their effect on cell survival. The design parameters of interest include pressure, flow rate, catheter diameter, catheter length, and cell concentration. Concentrations and survival rates of cells delivered from the settling prevention media are measured and compared to those of cells delivered from HBSS. Cells are allowed to settle for 40 minutes in an effort to determine whether cells suspended in settling prevention media can be delivered without the need for mixing.

### Example IV

Three separate delivery solutions were prepared. The cell concentration was held constant by using the ratio (2 mL cell suspension/1 mL carrier solution) for a total of 3 mL added to each of three delivery syringes. The cell suspension was the same in all three solutions. The makeup of each of the three carrier solutions was as follows:
Solutions 1 and 2: Hanks balanced salt solution (HBSS) as used in previous experiments
Solution 3: Isovue 370/deionized (DI) H₂O mix, adjusted for a specific gravity of 1.060 and an osmolality of 300 mOsm/kg
Solution 3 was prepared similarly to the cell settling prevention media of previous experiments, with the exception that Isovue 370 image enhancing media was used in place of Isovue 300. Isovue 370 is simply a more concentrated iopamidol solution than Isovue 300, and it was found that an additional dilution with DI H₂O (3.8 mL of DI H₂O for every 16.2 mL of Isovue 370) brought the properties of Isovue 370 media back to those of Isovue 300 media. Dilutions then continued as per the above referenced method in Example III.

Cells and carrier solutions were mixed in 50 mL centrifuge tubes, labeled Solutions 1, 2, and 3, and used as described below. Catheter assemblies 152.4 cm (60 inches) long were built from 0.305 mm (0.012 ") ID PEEK tubing. Myoblasts were stored in 50 mL centrifuge tubes throughout the experiment and were never frozen before use. Cells were delivered through catheter assemblies by use of the EFD Model 1500XL fluid delivery system. For each experiment, the following data were collected:
- Hemocytometer counts, and Trypan Blue viability staining, on myoblasts from each of the three initial 50 mL centrifuge tubes;
- Hemocytometer counts, and Trypan Blue viability staining, on myoblasts from each solution immediately after the (t=0 minutes) delivery through their respective catheters; and
- Hemocytometer counts, and Trypan Blue viability staining, on myoblasts from each solution immediately after the (t=40 minutes) delivery through their respective catheters.

Tables I, II, and III below show the mixing protocols for each solution. Briefly, Solution 1 is the HBSS/cells solution, mixed before t=0 but not before t=40; Solution 2 is the HBSS/cells solution, mixed before both t=0 and t=40; and Solution 3 is the Isovue/cells solution, mixed before t=0 but not before t=40.

**Table I**

| | |
|---|---|
| Solution # 1: | **HBSS, no mix at 40** |
| (t=0: 11:35 am; t=40: 12:15 pm) | |
| **1A** (t=0): | 4.30 M, 95% viable |
| **1B** (t=0): | 3.80 M, 94% viable |
| **1C** (t=0): | 4.30 M, 94% viable |
| **1D** (t=40): | 1.93 M, 92% viable |
| **1E** (t=40): | 1.43 M, 97% viable |
| **1F** (t=40): | 1.33 M, 95% viable |

**Table II**

| | |
|---|---|
| Solution #2: | **HBSS, mix at 40** |
| (t=0: 11:48 am; t=40: 12:28 pm) | |
| **2A** (t=0): | 4.20 M, 93% viable |
| **2B** (t=0): | 3.83 M, 93% viable |
| **2C** (t=0): | 3.83 M, 93% viable |
| **2D** (t=40): | 4.10 M, 91% viable |
| **2E** (t=40): | 3.98 M, 91% viable |
| **2F** (t=40): | 3.93 M, 88% viable |

**Table III**

| | |
|---|---|
| **Solution #3:** | **Isovue/HBSS, no mix at 40** |
| (t=0: 12:01 pm; t=40: 12:41 pm) | |
| **3A** (t=0): | 4.18 M, 97% viable |
| **3B** (t=0): | 4.03 M, 96% viable |
| **3C** (t=0): | 4.25 M, 96% viable |
| **3D** (t=40): | 3.75 M, 96% viable |
| **3E** (t=40): | 3.93 M, 93% viable |
| **3F** (t=40): | 4.00 M, 94% viable |

Canine skeletal myoblasts were cultured until sufficient cells were available. The myoblasts were dissociated, rinsed, and re-suspended in HBSS into a 50 mL centrifuge tube containing the appropriate carrier solution. In this experiment Applicants were able to deliver a minimum of I million cells/mL into the catheters.

Cells from all samples described in the preceding sections were manually counted in duplicate using a hemocytometer. Table IV shows the cell count ratios, with units in cells/mL.

**Table IV**

| Solution # | Delivery solution | Initial cell concentration | t=0 delivered cell concentration (average) | t=40 delivered cell concentration (average) | Ratio, t=0/initial (% of init) | **Ratio, t=40/t=0 (% of t=0)** |
|---|---|---|---|---|---|---|
| 1 | HBSS, no mix | 3.95 M | 4.13 M | 1.56 M | 104% | **38%** |
| 2 | HBSS, mix | 3.95 M | 3.95 M | 4.00 M | 100% | **101%** |
| 3 | Isovue, no mix | 4.47 M | 4.15 M | 3.89 M | 93% | **94%** |

The results of Example IV clearly demonstrate that use of a cell settling prevention media (in this case, a dilute solution of Isovue 370 media) allows for delivery of the initial concentration of myoblasts, even after 40 minutes without mixing have elapsed. The myoblast concentration after delivery from the settling prevention media is essentially unchanged after 40 minutes (94% of t=0 concentration), whereas significant numbers of myoblasts are lost after delivery following 40 minutes in HBSS without mixing (only 38% of the t=0 concentration was delivered). These results clearly show the effectiveness of cell settling prevention media for retaining myoblast concentrations in catheter delivery, even without mixing.

### Example V

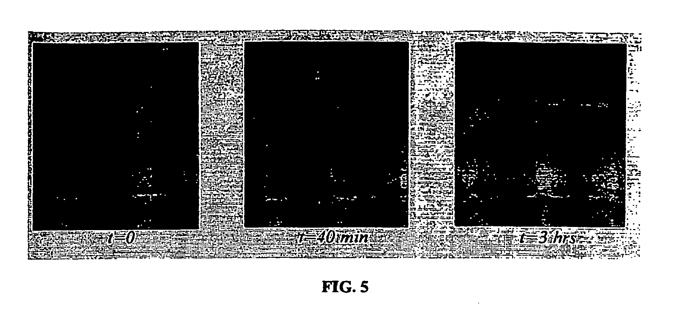

Human dermal fibroblasts were harvested, counted, and equally divided into two separate tubes. The number of cells in each tube was approximately 375 million cells. The makeup of each tube was as follows:
Solution -: Hanks balanced salt solution (HBSS) with cells
Solution + : Hanks balanced salt solution with cells mixed with Isovue 370, adjusted for a specific gravity of 1.060 and an osmolality of 300 mOsm/kg. The tubes were left at room temperature and pictures were taken at times 0, 40 minutes, and 3 hours (Figure 5). Figure 5 illustrates the effect that specific gravity matched solutions with Isovue 370 have, compared to normal Hanks balanced salt solutions on cell settling.

### Example VI

Several tests were made to evaluate the performance of cell delivery fluids across different catheter systems varying the materials, lengths, diameters, and delivery pressures (see also general catheter assemblies below) for different cell types (e.g., fibroblasts and myoblasts - see also cell preparation below) (Figure 6). Based on the various delivery parameters, e.g., shear rate, shear stress, shear time (see also fluid flow parameters below), percent of live cells resulting from delivery was measured.

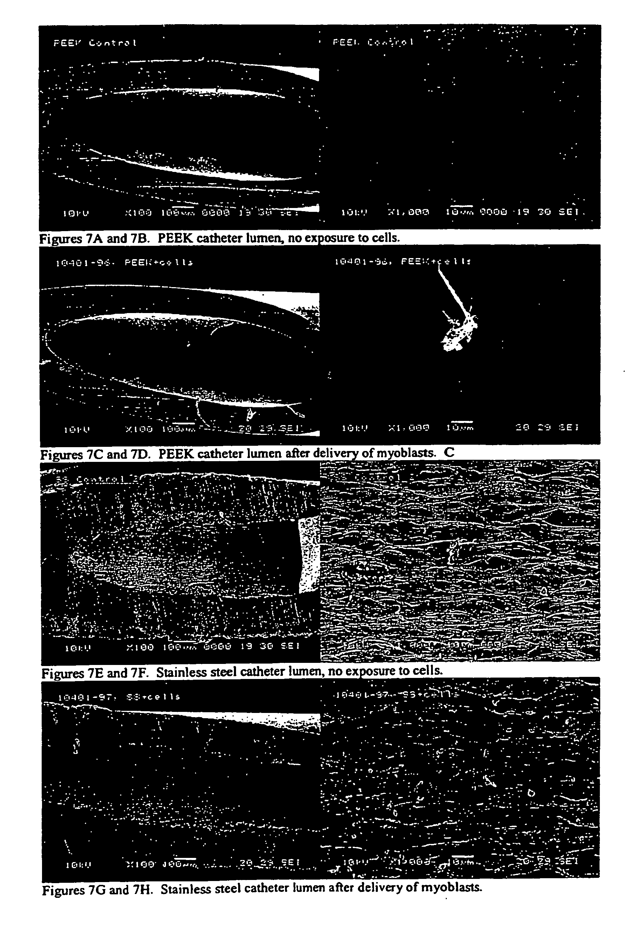

Figures 7A through 7H are SEM photographs of lumenal catheter surfaces. In each pair of figures, the image on the left was taken at 100X magnification, and the image on the right at 1000X. The 1000X images are representative areas from the approximate centers of the analogous 100X images: Figures 7A and 7B are photographs of PEEK catheter lumens after no exposure to cells; Figures 7C and 7D are photographs of PEEK catheter lumens after delivery of myoblasts; Figures 7E and 7F are photographs of stainless steel catheter lumens after no exposure to cells; Figures 7G and 7H are photographs of stainless steel catheter lumens after delivery of myoblasts . These images indicate that the cell delivery carriers left very few residual cells on the internal surfaces of the catheter.

### General Cell Preparation:

Human dermal fibroblasts, (Clonetics, Inc.) or, in later experiments, canine skeletal myoblasts, were cultured in tissue culture flasks using specialty growth media (Clonetics, Inc.). The media was replaced every three days and when confluent, the cells were passaged to propagate the cultures. After it was determined that sufficient cells were available, the cells were rinsed once with Hanks balanced salt solution (HBSS) and then dissociated with a 5 min enzymatic wash (0.25% trypsin) at 37°C. The resulting cell suspension was neutralized with serum containing growth media and then centrifuged (800g) for 10 min to pellet the cells. The supernatant was discarded and the pellet was resuspended in HBSS solution. At this point, the approximate cell concentration was determined by a hemocytometer cell count. The volume of HBSS was adjusted to obtain the desired cell concentration. The initial cell concentration, was calculated from the hemocytometer cell count and HBSS dilution. The final cell suspension was stored under ice for the duration of the experiment.

### General Test Catheter Assembly:

The test catheter assemblies were made by bonding segments of PEEK (polyetherether ketone), PI (polyimide), [or in later experiments, stainless steel (SS)] of various lengths and diameters to luer-lock stub adaptors with Loctite 401 adhesive after priming with Loctite 7701.

### General Fluid Flow Set-Up:

The fluid flow setup consisted of a fluid dispenser (EFD, Model 1500XL) driven by compressed air (max 586 kPa (85 psi)) fitted with a 3 cc syringe. The fluid to be dispensed (either the cell suspension of interest or DI water) was loaded into the syringe. The syringe tip was fitted with the test catheter assembly described in the previous section. Delivery time (to the nearest 0.1 second) and pressure (up to 552 kPa (80 psi)) can be fixed with this system. To ensure that a suitable volume of cell suspension was delivered, preliminary flow rate measurements were done with DI water.

It is recognized that any of various media may be suitable for the carrier medium, providing that it has the basic characteristics of density matching of intended cells for delivery, a known biologic compatibility, is preferably inexpensive, and is preferably suited for ionic salt solution-type of uses. The delivery device for a preferred cell delivery fluid may utilize components and techniques having a known look and feel to the physician and have ease of functionality due to the characteristics of the medium being that of a fluid with a low viscosity. The density matching technique identified by Applicants permits a more simplified structure of delivery system by obviating the need for complex mixing zones, leuers, or the like. With the cells evenly distributed in all the vessels and tubing of the delivery system, an accurate and consistent number of cells will be delivered- achieving a more consistent therapeutic result. This is achievable without requiring mixing or vibrating the fluid after placement into the delivery catheter. In one embodiment, a catheter is a disposable medical device, although this is not required. A delivery system catheter may be constructed from medical grade plastics and/or other materials, such as stainless steel or other suitable material.

Another embodiment of this invention includes a method of increasing the efficacy of cell delivery to a patient comprising the steps of providing a carrier liquid for delivering accurate concentrations of cells into a patient. The carrier liquid should have a density which substantially matches the density of the cells to be delivered into the patient when mixed in solution. Also, the solution should have a pH in the range of about 6.0 to 8.0, and more preferably 6.8 to 7.6, and most preferrably about 7.0, about 7.2, and about 7.4. Preferrably the balanced medium is substantially isotonic as previously described. A further step involves combining the cell delivery of density matched solution of carrier liquid and cells with another medical procedure. The medical procedure may be of various types, and for virtually any medical application. Although the combination may occur at the same time, the combination might not be accomplished simultaneously but rather in a synergistic manner to increase the efficacy of one or both of the cell delivery and the other medical procedure. In one embodiment the medical procedure may be selected from the list of cardiac pacing, cardiac stimulation, cardiac electrical therapy, cardiac pharmacologic therapy, cardiac monitoring, cardiac imaging, cardiac sensing, cardiac mapping, interventional procedures, surgical procedures, infusion procedures, diagnostic procedures, and therapeutic procedures. In another embodiment the medical procedure may be selected from the list of neurologic stimulation, neurologic electrical therapy, neurologic pharmacologic therapy, neurologic monitoring, neurologic imaging, neurologic sensing, neurologic mapping, interventional procedures, surgical procedures, infusion procedures, diagnostic procedures, and therapeutic procedures.

Thus, embodiments of a cell delivery fluid for prevention of cell settling in delivery systems are disclosed. One skilled in the art will appreciate that the present invention can be practiced with embodiments other than those disclosed. The disclosed embodiments are presented for purposes of illustration and not limitation, and the present invention is limited only by the claims which follow.

## Claims

1. A method of preparing a cell-carrier liquid suspension for implantation into a mammal, comprising:
a. selecting a type of cell for implantation into a mammal and identifying the specific gravity of the cell type;
b. selecting a carrier liquid having a specific gravity within a range of specific gravities designed to approximately match the specific gravity of the selected cell type to produce an acceptable delivery ratio of cells at a delivery destination in the mammal;
c. ensuring that the identified liquid has an appropriate osmolality and pH to ensure an acceptable viability of the cells at the delivery destination; and
d. mixing the carrier liquid and the cells into liquid suspension.

2. The method of claim 1 in which the cell selection step comprises selecting from types of cells which include fibroblasts or myoblasts.

3. The method of claim 1 in which the cell selection step comprises selecting a type of cell from the group consisting of islet cells, pluripotent stem cells, mesenchymal stem cells, endodermal stem cells, ectodermal stem cells, hepatocytes, chondrocytes, osteoblasts, neuronal cells, glial cells, smooth muscle cells, endothelial cells, skeletal myoblasts, nucleus pulposus cells, epithelial cells, myoblast, alpha islet cells, beta islet cells, macrophages, cardiomyocytes, purkinje cells, erythrocytes, platelets and fibroblasts.

4. The method of claim 1 in which the carrier liquid selecting step comprises selecting a liquid which has a specific gravity that is within the group selected from 5%, 2%, 1 %, 0.1% and 0.01% of the specific gravity of the cell type.

5. The method of claim 1 in which the cell type selection step further includes placing the selected cell type into a solution.

6. The method of claim 1 in which the mixing step results in a substantially isotonic liquid suspension.

7. The method of claim 1 in which the mixing step results in a liquid suspension that is within +300/-50 mOsm/kg of isotonic.

8. The method of claim 1 in which the step of ensuring that the identified liquid has an appropriate osmolality and pH ensures that the delivered cells are at least 80% viable for a period of at least 4 hours.

9. The method of claim 1 in which the step of ensuring that the identified liquid has an appropriate pH comprises selection of a liquid having a pH in the range of 6.0 to 8.0.

10. The method of claim 1 in which the mixing step comprises mixing the carrier liquid and the cells in a bioreactor.

11. The method of claim 1 in which the cells have been labeled or tagged with a cell identification marker.

12. The method of claim 1 in which the cell carrier liquid also contains an image enhancing agent.

13. The method of claim 1 in which the cell carrier liquid is also an image enhancing agent.

14. The method of claim 1 in which the cell carrier liquid also contains a radioactive element.

15. A cell delivery carrier liquid suspension for delivery of cells into a tissue of a mammal, comprising:
a. a volume of cells for delivery to a destination site in a mammal to generate new tissue growth;
b. a carrier liquid having a density which substantially matches the density of the cells when mixed in solution, and the solution having a pH in the range of 6.0 to 8.0, and being substantially isotonic;
c. wherein the cells remain substantially dispersed in the carrier liquid solution to provide a consistent delivery concentration of cells to the destination site.

16. The suspension of claim 15, in which the cells are selected from the group consisting of cells that form cartilage, cells that form bone, muscle cells, fibroblasts, and organ cells.

17. The suspension of claim 15 in which the osmolality of the suspension is 300 mOsm/kg.

18. The suspension of claim 15 in which the cells are selected from the group consisting of islet cells, pluripotent stem cells, mesenchymal stem cells, endodermal stem cells, ectodermal stem cells, hepatocytes, chondrocytes, osteoblasts, neuronal cells, glial cells, smooth muscle cells, endothelial cells, skeletal myoblasts, nucleus pulposus cells, epithelial cells, myoblast, alpha islet cells, beta islet cells, macrophages, cardiomyocytes, purkinje cells, erythrocytes, platelets, and fibroblasts.

19. The suspension of claim 15 in which the carrier liquid comprises a formulation selected from the group of iopamidol, perfluorooctyl bromide, gadodiamide, and iron dextrans.

20. The suspension of claim 15 in which the cells have been labeled or tagged with a cell identification marker.

21. The suspension of claim 15 in which the cell carrier liquid also contains an image enhancing agent.

22. The suspension of claim 15 in which the cell carrier liquid is also an image enhancing agent.

23. The suspension of claim 15 in which the cell carrier liquid also contains a radioactive element.

24. The use of cells for the manufacture of a pharmaceutical composition for the treatment of myocardial infarctions or congestive heart failure, said use comprising the steps of
a) providing a carrier liquid for delivering accurate concentration of cells, the carrier liquid having a density which substantially matches the density of the cells to be delivered into the patient when mixed in solution, and the solution having a pH in the range of 6.0 to 8.0, and being substantially isotonic; and
b) wherein administration of density matched solution of carrier liquid and cells is to be combined with another medical procedure selected from the list of cardiac pacing, cardiac stimulation, cardiac electrical therapy, cardiac pharmacologic therapy, cardiac monitoring, cardiac imaging, cardiac sensing, cardiac mapping and procedures relating to the cardiovascular system.

25. The use of claim 24 in which the cell delivery is to be accomplished with a pump.

26. The use of claim 24 in which the cell delivery is to be accomplished with a catheter.

27. The use of claim 24 in which the cell delivery is to be accomplished with a syringe.

28. The use of claim 24 in which the cell delivery is to be accomplished in combination with a drug infusion mechanism.

29. The use of claim 24 in which the cell carrier liquid is also an image enhancing gent.

30. The use of claim 24 in which the cell carrier liquid also contains a radioactive element.

31. The use of claim 24 in which the cells are selected from the list of islet cells, pluripotent stem cells, mesenchymal stem cells, endodermal stem cells, ectodermal stem cells, hepatocytes, chondrocytes, osteoblasts, neuronal cells, glial cells, smooth muscle cells, endothelial cells, skeletal myoblasts, nucleus pulposus cells, epithelial cells, myoblast, alpha islet cells, beta islet cells, macrophages, cardiomyocytes, purkinje cells, erythrocytes, platelets, and fibroblasts.

32. The use of claim 24 in which the cells have been labeled or tagged with a cell identification marker.

33. The use of claim 24 in which the cell carrier liquid also contains an image enhancing agent.

34. A new use for a liquid having certain characteristics suitable for use or adjustment and use as a cell delivery carrier medium, in which the new use comprises:
a. configuring the liquid as a carrier liquid having a density which substantially matches the density of the cells to be delivered to a mammalian tissue growth site, the liquid being configured so that when it is mixed as a solution with the cells, then the solution has a pH in the rage of 6.0 to 8.0, and is substantially isotonic;
b. wherein the cells remain substantially dispersed in the carrier liquid solution to provide a consistent delivery concentration of cells to the destination site.

35. The use for the liquid of claim 34 in which the liquid comprises a formulation selected from the group of non-ionic image enhancing agents.

36. The use for the liquid of claim 34 in which the liquid comprises a formulation selected from the group of iopamidol and perfluorooctyl bromide.

37. The use for the liquid of claim 34 in which the cells have been labeled or tagged with a cell identification marker.

38. The use for the liquid of claim 34 in which the cell carrier liquid is also an image enhancing agent.

39. The use for the liquid of claim 34 in which the cell carrier liquid also contains a radioactive element.

40. The cell delivery carrier liquid of any of claims 1,15,24 or 34 in which the carrier liquid selecting step comprises selecting a liquid which has a specific gravity that is within the group selected from 5%, 2%, 1%, 0.1% and 0.01 % of a cell-specific gravity of 1.063 to 1.10.

## Patentansprüche

1. Verfahren zum Vorbereiten einer Zell-Träger- bzw. Carrier-Liquidsuspension zur Implantation in ein Säugetier bzw. einen Säuger, umfassend:
a) Auswählen eines Zelltyps zur Implantation in ein Säugetier und Identifizieren des spezifischen Gewichts des Zelltyps;
b) Auswählen eines Carrier-Liquids mit einem spezifischen Gewicht innerhalb eines Bereichs von spezifischen Gewichten, die dazu ausgestaltet sind, ungefähr zu dem spezifischen Gewicht des ausgewählten Zelltyps zu passen, um ein annehmbares Abgabeverhältnis der Zellen an einem Abgabeziel in dem Säugetier zu erreichen bzw. zu erzeugen;
c) Sicherstellen, dass das identifizierte Liquid eine geeignete Osmolalität und einen geeigneten pH-Wert aufweist, um eine annehmbare Lebensfähigkeit der Zellen am Abgabeziel sicherzustellen; und
d) Mischen des Carrier-Liquids und der Zellen zu einer Liquidsuspension.

2. Verfahren nach Anspruch 1, bei dem der Zellauswahlschritt das Auswählen aus Zelltypen umfasst, die Fibroblasten oder Myoblasten einschließen.

3. Verfahren nach Anspruch 1, bei dem der Zellauswahlschritt das Auswählen eins Zelltyps aus der Gruppe umfasst, die aus Inselzellen, pluripotenten Stammzellen, mesenchymalen Stammzellen, endodermalen Stammzellen, ektodermalen Stammzellen, Leberzellen, Chondrozyten, Osteoblasten, Neuronalzellen, Gliazellen, glatten Muskelzellen, Endothelzellen, Skelettmyoblasten, nucleus pulposus Zellen, Epithelzellen, Myoblasten, Alphainselzellen, Betainselzellen, Makrophagen, Kardiomyozyten, Purkinje-Zellen, Erythrozyten, Thrombozyten und Fibroblasten besteht.

4. Verfahren nach Anspruch 1, bei dem der Carrier Liquid Auswahlschritt das Auswählen eines Liquids umfasst, welches ein spezifisches Gewicht aufweist, das innerhalb der Gruppe ausgewählt aus 5 %, 2%, 1%, 0,1 % und 0,01 % des spezifischen Gewichts des Zelltyps liegt.

5. Verfahren nach Anspruch 1, bei dem der Zelltypauswahlschritt ferner das Anordnen des ausgewählten Zelltyps in einer Lösung umfasst.

6. Verfahren nach Anspruch 1, bei dem der Mischschritt in einer im wesentlichen isotonischen Liquidsuspension resultiert.

7. Verfahren nach Anspruch 1, bei dem der Mischschritt in einer Liquidsuspension resultiert, die innerhalb +300/-50 mOsm/kg isotonisch ist.

8. Verfahren nach Anspruch 1, bei dem der Schritt des Sicherstellens, dass das identifizierte Liquid eine geeignete Osmolalität und einen geeigneten pH-Wert aufweist, sicherstellt, dass die abgegebenen Zellen zumindest 80 % lebensfähig über eine Zeitspanne von wenigstens vier Stunden sind.

9. Verfahren nach Anspruch 1, bei dem der Schritt des Sicherstellens, dass das identifizierte Liquid einen geeigneten pH aufweist, das Auswählen eines Liquids mit einem pH im Bereich von 6,0 bis 8,0 umfasst.

10. Verfahren nach Anspruch 1, bei dem der Mischschritt das Mischen des Carrier-Liquids und der Zellen in einem Bioreaktor umfasst.

11. Verfahren nach Anspruch 1, bei dem die Zellen beschriftet oder mit einer Zellidentifikationsmarkierung markiert worden sind.

12. Verfahren nach Anspruch 1, bei dem das Zell-Carrier-Liquid auch einen Kontrasterhöhungswirkstoff enthält.

13. Verfahren nach Anspruch 1, bei dem das Zell-Carrier-Liquid auch ein Kontrasterhöhungswirkstoff ist.

14. Verfahren nach Anspruch 1, bei dem das Zell-Carrier-Liquid auch ein radioaktives Element enthält.

15. Zellabgabe-Carrier-Liquidsuspension zur Abgabe von Zellen in ein Gewebe eines Säugetiers mit:
a) Einem Volumen von Zellen zur Abgabe an eine Zielstelle in einem Säugetier bzw. Säuger, um neues Zellwachstum zu generieren;
b) einem Carrier-Liquid mit einer Dichte, die im wesentlichen zu der Dichte der Zellen, wenn sie in der Lösung gemischt sind, passt, und wobei die Lösung einen pH-Wert im Bereich von 6,0 bis 8,0 aufweist, und wobei es im wesentlichen isotonisch ist;
c) wobei die Zellen in der Carrier-Liquid-Lösung im wesentlichen dispergiert bleiben, um eine konsistente Abgabekonzentration der Zellen an die Zielstelle bereitzustellen.

16. Suspension nach Anspruch 15, bei der die Zellen aus der Gruppe ausgewählt sind, die aus knorpelbildenden Zellen, knochenbildenden Zellen, Muskelzellen, Fibroblasten und organischen Zellen besteht.

17. Suspension nach Anspruch 15, bei der die Osmolalität der Suspension 300 mOsm/kg ist.

18. Suspension nach Anspruch 15, bei der die Zellen aus der Gruppe ausgewählt sind, die aus Inselzellen, pluripotenten Stammzellen, mesenchymalen Stammzellen, endodermalen Stammzellen, ektodermalen Stammzellen, Leberzellen, Chondrozyten, Osteoblasten, Neuronalzellen, Gliazellen, glatten Muskelzellen, Endothelzellen, Skelettmyoblasten, nucleus pulposus Zellen, Epithelzellen, Myoblasten, Alphainselzellen, Betainselzellen, Makrophagen, Kardiomyozyten, Purkinje-Zellen, Erythrozyten, Thrombozyten und Fibroblasten besteht.

19. Suspension nach Anspruch 15, bei der das Carrier-Liquid eine Formel aufweist, die aus der Gruppe von Iopamidol, Perfluorooctylbromid, Gadodiamid und Eisendextran ausgewählt ist.

20. Suspension nach Anspruch 15, bei der die Zellen beschriftet oder mit einer Zellidentifikationsmarkierung markiert sind.

21. Suspension nach Anspruch 15, bei der das Zell-Carrier-Liquid auch einen Kontrasterhöhungswirkstoff enthält.

22. Suspension nach Anspruch 15, bei der das Zell-Carrier-Liquid auch ein Kontrasterhöhungswirkstoff ist.

23. Suspension nach Anspruch 15, bei der das Zell-Carrier-Liquid auch ein radioaktives Element enthält.

24. Verwendung von Zellen für die Herstellung einer pharmazeutischen Zusammensetzung für die Behandlung von Myokardinfarkten oder Herzdekompensation, wobei die Verwendung die Schritte umfasst:
a) Bereitstellen eines Carrier-Liquids zur Abgabe einer genauen Konzentration von Zellen, wobei das Carrier-Liquid eine Dichte aufweist, die im wesentlichen zu der Dichte der Zellen, die an den Patienten abgegeben werden sollen, wenn sie in einer Lösung gemischt sind, passt, und wobei die Lösung einen pH-Wert im Bereich von 6,0 bis 8,0 aufweist, und wobei es im wesentlichen isotonisch ist; und
b) wobei die Anwendung bzw. Verabreichung der Lösung des Carrier-Liquids und der Zellen mit der passende Dichte mit einem anderen medizinischen Verfahren zu kombinieren ist, das aus der Liste Herzschrittmachung, Herzstimulation, elektrische Herztherapie, pharmokologische Herztherapie, Herzüberwachung, Herzbildgebung, Herzerfassung, Herzkartographie und Verfahren, die mit dem kardiovaskulären System zusammenhängen, ausgewählt ist.

25. Verwendung nach Anspruch 24, bei der die Zellabgabe mit einer Pumpe erfolgt.

26. Verwendung nach Anspruch 24, bei der die Zellabgabe mit einem Katheter erfolgt.

27. Verwendung nach Anspruch 24, bei der die Zellabgabe mit einer Spritze erfolgt.

28. Verwendung nach Anspruch 24, bei der die Zellabgabe in Kombination mit einem Medikamenteninfusionsmechanismus erfolgt.

29. Verwendung nach Anspruch 24, bei der das Zell-Carrier-Liquid auch ein Kontrasterhöhungswirkstoff ist.

30. Verwendung nach Anspruch 24, bei der das Zell Carrier Liquid auch ein radioaktives Element enthält.

31. Verwendung nach Anspruch 24, bei der die Zellen aus der Liste von pluripotenten Stammzellen, mesenchymalen Stammzellen, endodermalen Stammzellen, ektodermalen Stammzellen, Leberzellen, Chondrozyten, Osteoblasten, Neuronalzellen, Gliazellen, glatten Muskelzellen, Endothelzellen, Skelettmyoblasten, nucleus pulposus Zellen, Epithelzellen, Myoblasten, Alphainselzellen, Betainselzellen, Makrophagen, Kardiomyozyten, Purkinje-Zellen, Erythrozyten, Thrombozyten und Fibroblasten ausgewählt sind.

32. Verwendung nach Anspruch 24, bei der die Zellen beschriftet oder mit einer Zellidentifikationsmarkierung markiert sind.

33. Verwendung nach Anspruch 24, bei der das Zell-Carrier-Liquid auch einen Kontrasterhöhungswirkstoff enthält.

34. Neue Verwendung für ein Liquid mit gewissen Eigenschaften, das für die Verwendung oder Einstellung und Verwendung als Zellabgabe-Carrier-Medium geeignet ist, wobei die neue Verwendung umfasst:
a) Konfigurieren des Liquids als ein Carrier-Liquid mit einer Dichte, welche im wesentlichen zu der Dichte der Zellen, die an eine Säugetiergewebewachstumsstelle abgegeben werden sollen, passt, wobei das Liquid konfiguriert ist, so dass, wenn es als Lösung mit den Zellen gemischt ist, dann die Lösung einen pH im Bereich von 6,0 bis 8,0 aufweist, und es im wesentlichen isotonisch ist;
b) wobei die Zellen im wesentlichen in der Carrier-Liquid-Lösung dispergiert bleiben, um eine konsistente Abgabekonzentration der Zellen an die Zielstelle bereitzustellen.

35. Verwendung für das Liquid nach Anspruch 34, bei der das Liquid eine Formel aufweist, die aus der Gruppe von nicht-ionischen Kontrasterhöhungswirkstoffen ausgewählt ist.

36. Verwendung für das Liquid nach Anspruch 34, bei der das Liquid eine Formel aufweist, die aus der Gruppe von Iopamidol und Perfluorooctylbromid ausgewählt ist.

37. Verwendung für das Liquid nach Anspruch 34, bei der die Zellen beschriftet oder mit einer Zellidentifikationsmarkierung markiert sind.

38. Verwendung für das Liquid nach Anspruch 34, bei der das Zell-Carrier-Liquid auch ein Kontrasterhöhungswirkstoff ist.

39. Verwendung für das Liquid nach Anspruch 34, bei der das Zell-Carrier-Liquid auch ein radioaktives Element enthält.

40. Zellabgabe-Carrier-Liquid nach einem der Anspruche 1, 15, 24 oder 34, bei dem der Carrier-Liquid-Auswahlschritt das Auswählen eines Liquids umfasst, welches ein spezifisches Gewicht aufweist, das innerhalb der Gruppe ausgewählt aus 5 %, 2 %, 1 %, 0,1 % und 0,01 % eines zellspezifischen Gewichts von 1,063 bis 1,10 liegt.

## Revendications

1. Procédé pour préparer une suspension liquide de support cellulaire pour une implantation dans un mammifère, comportant :
a. la sélection d'un type de cellule pour une implantation dans un mammifère et l'identification de la gravité spécifique du type cellulaire,
b. la sélection d'un liquide de support ayant une gravité spécifique dans une plage de gravités spécifiques conçue pour correspondre approximativement à la gravité spécifique du type de cellule sélectionné pour produire un rapport acceptable de libération de cellules au niveau d'une destination de libération dans le mammifère,
c. l'assurance que le liquide identifié a une osmolalité et un pH appropriés pour garantir une viabilité acceptable des cellules au niveau de la destination de libération, et
d. le mélange du liquide de support et des cellules dans une suspension liquide.

2. Procédé selon la revendication 1, dans lequel l'étape de sélection de cellule comporte la sélection à partir de types de cellules qui incluent des fibroblastes ou myoblastes.

3. Procédé selon la revendication 1, dans lequel l'étape de sélection de cellule comporte la sélection d'un type de cellule parmi le groupe constitué d'îlots de Langerhans, cellules souche pluripotentes, cellules souche mésenchymales, cellules souche endodermiques, cellules souche ectodermiques, hépatocytes, chondrocytes, ostéoblastes, cellules neuronales, cellules gliales, cellules de muscle lisse, cellules endothéliales, myoblastes squelettiques, cellules du nucleus pulposus, cellules épithéliales, myoblaste, îlot alpha de Langerhans, îlot bêta de Langerhans, macrophages, cardiomyocytes, cellules de Purkinje, érythrocytes, plaquettes et fibroblastes.

4. Procédé selon la revendication 1, dans lequel l'étape de sélection de liquide de support comporte la sélection d'un liquide qui a une gravité spécifique qui se trouve dans le groupe sélectionné parmi 5 %, 2 %, 1 %, 0,1 % et 0,01 % de la gravité spécifique du type de cellule.

5. Procédé selon la revendication 1, dans lequel l'étape de sélection de type de cellule inclut de plus l'introduction du type de cellule sélectionné dans une solution.

6. Procédé selon la revendication 1, dans lequel l'étape de mélange a pour résultat une suspension liquide essentiellement isotonique.

7. Procédé selon la revendication 1, dans lequel l'étape de mélange a pour résultat une suspension liquide qui se trouve dans la plage de +300/-50 mOsm/kg de suspension isotonique.

8. Procédé selon la revendication 1, dans lequel l'étape consistant à garantir que le liquide identifié a une osmolalité et un pH appropriés garantit que les cellules libérées sont viables à au moins 80 % sur une période d'au moins 4 heures.

9. Procédé selon la revendication 1, dans lequel l'étape consistant à garantir que le liquide identifié a un pH approprié comporte la sélection d'un liquide ayant un pH dans la plage de 6,0 à 8,0.

10. Procédé selon la revendication 1, dans lequel l'étape de mélange comporte le mélange du liquide de support et des cellules dans un bioréacteur.

11. Procédé selon la revendication 1, dans lequel les cellules ont été étiquetées ou marquées à l'aide d'un marqueur d'identification cellulaire.

12. Procédé selon la revendication 1, dans lequel le liquide de support cellulaire contient également un agent accentuateur d'image.

13. Procédé selon la revendication 1, dans lequel le liquide de support cellulaire est également un agent accentuateur d'image.

14. Procédé selon la revendication 1, dans lequel le liquide de support cellulaire contient également un élément radioactif.

15. Suspension liquide de support de libération cellulaire pour libérer des cellules dans un tissu d'un mammifère, comportant :
a. une volume de cellules pour une libération dans un site de destination chez un mammifère pour générer une nouvelle croissance tissulaire,
b. un liquide de support ayant une densité qui correspond essentiellement à la densité des cellules lorsque mélangées en solution, et la solution ayant un pH dans la plage de 6,0 à 8,0, et étant essentiellement isotonique,
c. dans lequel les cellules restent essentiellement dispersées dans la solution liquide de support pour fournir une concentration de libération uniforme de cellules dans le site de destination.

16. Suspension selon la revendication 15, dans laquelle les cellules sont sélectionnées parmi le groupe constitué de cellules qui forment un cartilage, de cellules qui forment un os, de cellules musculaires, des fibroblastes, et de cellules organiques.

17. Suspension selon la revendication 15, dans laquelle l'osmolalité de la suspension est de 300 mOsm/kg.

18. Suspension selon la revendication 15, dans laquelle les cellules sont sélectionnées parmi le groupe constitué d'îlots de Langerhans, cellules souche pluripotentes, cellules souche mésenchymales, cellules souche endodermiques, cellules souche ectodermiques, hépatocytes, chondrocytes, ostéoblastes, cellules neuronales, cellules gliales, cellules de muscle lisse, cellules endothéliales, myoblastes squelettiques, cellules du nucleus pulposus, cellules épithéliales, myoblaste, îlots alpha de Langerhans, îlots bêta de Langerhans, macrophages, cardiomyocytes, cellules de Purkinje, érythrocytes, plaquettes, et fibroblastes.

19. Suspension selon la revendication 15, dans laquelle le liquide de support comporte une formulation sélectionnée parmi le groupe constitué d'iopamidol, bromure de perfluorooctyle, gadodiamide, et fer dextrans.

20. Suspension selon la revendication 15, dans laquelle les cellules sont été étiquetées ou marquées à l'aide d'un marqueur d'identification cellulaire.

21. Suspension selon la revendication 15, dans laquelle le liquide de support cellulaire contient également un agent accentuateur d'image.

22. Suspension selon la revendication 15, dans laquelle le liquide de support cellulaire est également un agent accentuateur d'image.

23. Suspension selon la revendication 15, dans laquelle le liquide de support cellulaire contient également un élément radioactif.

24. Utilisation de cellules pour la fabrication d'une composition pharmaceutique destinée au traitement d'infarctus du myocarde ou d'insuffisance cardiaque congestive, ladite utilisation comportant les étapes consistant à :
a) fournir un liquide de support pour délivrer une concentration précise de cellules, le liquide de support ayant une densité qui correspond essentiellement à la densité des cellules à libérer dans le patient lorsque mélangées en solution, et la solution ayant un pH dans la plage de 6,0 à 8,0, et étant essentiellement isotonique, et
b) dans lequel l'administration d'une solution à densité correspondante de liquide de support et de cellules doit être combinée à un autre procédé médical sélectionné parmi la liste suivante : un entraînement cardiaque, stimulation cardiaque, thérapie électrique cardiaque, thérapie pharmacologique cardiaque, surveillance cardiaque, imagerie cardiaque, détection cardiaque, cartographie cardiaque et des procédés associés au système cardiovasculaire.

25. Utilisation selon la revendication 24, dans laquelle la libération cellulaire est à effectuer à l'aide d'une pompe.

26. Utilisation selon la revendication 24, dans laquelle la libération cellulaire est à effectuer à l'aide d'un cathéter.

27. Utilisation selon la revendication 24, dans laquelle la libération cellulaire est à effectuer à l'aide d'une seringue.

28. Utilisation selon la revendication 24, dans laquelle la libération cellulaire est à effectuer en combinaison avec un mécanisme de perfusion de médicament.

29. Utilisation selon la revendication 24, dans laquelle le liquide de support cellulaire est également un agent accentuateur d'image.

30. Utilisation selon la revendication 24, dans laquelle le liquide de support cellulaire contient également un élément radioactif.

31. Utilisation selon la revendication 24, dans laquelle les cellules sont sélectionnées parmi la liste suivante : îlots de Langerhans, cellules souche pluripotentes, cellules souche mésenchymales, cellules souche endodermiques, cellules souche ectodermiques, hépatocytes, chondrocytes, ostéoblastes, cellules neuronales, cellules gliales, cellules de muscle lisse, cellules endothéliales, myoblastes squelettiques, cellules du nucleus pulposus, cellules épithéliales, myoblaste, îlots alpha de Langerhans, îlots bêta de Langerhans, macrophages, cardiomyocytes, cellules de Purkinje, érythrocytes, plaquettes, et fibroblastes.

32. Utilisation selon la revendication 24, dans laquelle les cellules ont été étiquetées ou marquées à l'aide d'un marqueur d'identification cellulaire.

33. Utilisation selon la revendication 24, dans laquelle le liquide de support cellulaire contient également un agent accentuateur d'image.

34. Nouvelle utilisation pour un liquide ayant certaines caractéristiques adaptées à une utilisation ou un ajustement et une utilisation en tant que milieu de support de libération cellulaire, dans laquelle la nouvelle utilisation comporte :
a. la configuration du liquide en tant que liquide de support ayant une densité qui correspond essentiellement à la densité des cellules à libérer dans un site de croissance tissulaire de mammifère, le liquide étant configuré de sorte que, lorsqu'il est mélangé sous forme d'une solution avec les cellules, alors la solution a un pH dans la plage de 6,0 à 8,0, et est essentiellement isotonique,
b. dans laquelle les cellules restent essentiellement dispersées dans la solution liquide de support pour fournir une concentration de libération uniforme des cellules dans le site de destination.

35. Utilisation pour le liquide selon la revendication 34, dans laquelle le liquide comporte une formulation sélectionnée parmi le groupe d'agents accentuateurs d'image non-ioniques.

36. Utilisation pour le liquide selon la revendication 34, dans laquelle le liquide comporte une formulation sélectionnée parmi le groupe constitué d'iopamidol et de bromure de perfluorooctyle.

37. Utilisation pour le liquide selon la revendication 34, dans laquelle les cellules ont été étiquetées ou marquées à l'aide d'un marqueur d'identification cellulaire.

38. Utilisation pour le liquide selon la revendication 34, dans laquelle le liquide de support cellulaire est également un agent accentuateur d'image.

39. Utilisation pour le liquide selon la revendication 34, dans laquelle le liquide de support cellulaire contient également un élément radioactif.

40. Liquide de support de libération cellulaire selon l'une quelconque des revendications 1, 15, 24 ou 34, dans lequel l'étape de sélection de liquide de support comporte la sélection d'un liquide qui a une gravité spécifique qui se trouve dans le groupe sélectionné parmi 5 %, 2 %, 1 %, 0,1 % et 0,01 % d'une gravité cellulaire spécifique de 1,063 à 1,10.
